# EUROPEAN PATENT SPECIFICATION

(11) **EP 3 004 038 B1**
(45) Date of publication and mention of the grant of the patent: **29.08.2018**
(21) Application number: 14726655.5
(22) Date of filing: 28.05.2014
(51) Int. Cl.: C07C 29/86, C07C 31/20

(54) **PROCESS FOR THE SEPARATION OF ALCOHOLS**
VERFAHREN ZUR TRENNUNG VON ALKOHOLEN
PROCÉDÉ DE SÉPARATION D'ALCOOLS

(30) Priority: 31.05.2013 EP 13169977
(43) Date of publication of application: 13.04.2016
(73) Proprietor: Shell Internationale Research Maatschappij B.V., 2596 HR The Hague (NL)
(72) Inventor: FISCHER, Kai, Jürgen, NL-1031HW Amsterdam (NL); KOOT, Wouter, NL-1031 HW Amsterdam (NL); BUS, Karin, NL-1031 HW Amsterdam (NL); HUIZENGA, Pieter, NL-1031 HW Amsterdam (NL)
(74) Representative: Shell Legal Services IP
(86) International application number: PCT/EP2014/061169
(87) International publication number: WO 2014/191516

(56) References cited:
- EP-A2- 0 844 228
- WO-A1-2012/130316
- US-A- 2 831 801
- US-A1- 2012 184 783

## Description

### Field of the Invention

The present invention relates to a process for the separation of glycols.

### Background of the Invention

Ethylene glycol and propylene glycol are valuable materials with a multitude of commercial applications, e.g. as heat transfer media, antifreeze, and precursors to polymers, such as PET. Ethylene and propylene glycols are typically made on an industrial scale by hydrolysis of the corresponding alkylene oxides, which are the oxidation products of ethylene and propylene, produced from fossil fuels.

In recent years, increased efforts have focused on producing chemicals, including glycols, from renewable feedstocks, such as sugar-based materials. For example, US 2011/312050 describes a continuous process for the catalytic generation of polyols from cellulose, in which the cellulose is contacted with hydrogen, water and a catalyst to generate an effluent stream comprising at least one polyol.

CN 102643165 is directed to a catalytic process for reacting sugar in an aqueous solution with hydrogen in the presence of a catalyst in order to generate polyols.

As with many chemical processes, the reaction product stream in these reactions comprises a number of desired materials, diluents, by-products and other undesirable materials. In order to provide a high value process, the desirable product or products must be obtainable from the reaction product stream in high purity with a high percentage recovery of each product and with as low as possible use of energy and complex equipment.

In known processes to make glycols, the glycols are usually present at high dilution in a solvent, typically water. The water is usually removed from the glycols by distillation. Subsequent purification of the glycols is then carried out by fractional distillation. This process can have high costs both in terms of capital and operational expenditure. Further, repeated heating or maintenance at raised temperatures in the distillation steps can also lead to decomposition of the desired glycol products.

When glycols are produced by hydrogenolysis of sugars, a mixture of glycols is produced. The main glycol constituents in the reaction product stream are monoethylene glycol (MEG), monopropylene glycol (MPG) and 1,2-butanediol (1,2-BDO). The separation of these glycols by fractional distillation is problematic due to the similarity in boiling points, particularly between MEG and 1,2-BDO.

US 2012/184783 is directed to an extraction process for recovery of purified ethylene glycol from a mixed diol stream in which the ethylene glycol is recovered from a raffinate phase leaving a mixture of other diols and a minor amount of ethylene glycol in the extract phase.

It would, therefore, be advantageous to provide an improved method suitable for the recovery of individual alcohols, particularly glycols, from mixture of alcohols.

### Summary of the Invention

Accordingly, the present invention provides a process for the recovery of a first glycol from a stream comprising two or more glycols, said process comprising the steps of providing a stream comprising two or more glycols, providing a solvent stream, combining said stream comprising two or more glycols with said solvent stream in the presence of water and recovering at least a portion of the first glycol by liquid-liquid extraction, wherein the first glycol is recovered from the solvent of the solvent stream and the solvent comprises an alkylamine.

### Brief Description of the Drawings

Figures 1 and 2 are schematic diagrams of exemplary, but non-limiting, embodiments of a process for the separation of alcohols as described herein.
Figure 3 illustrates the selectivities of glycols over water at three temperatures, 20, 50 and 90 °C.

### Detailed Description of the Invention

The present inventors have surprisingly found that glycols may be individually and independently recovered from a stream comprising a mixture of glycols by the use of a solvent in liquid-liquid extraction.

The present invention provides a process for the recovery of a first glycol from a stream comprising two or more glycols. The stream comprising two or more glycols may be an aqueous stream comprising in the range of from 0.1 to 100wt% water or it may be a stream comprising two or more glycols and only trace amounts of water, or less, including no water.

Preferably, the stream comprising two or more alcohols is the reaction product stream from a process for the production of alcohols. In a particularly preferred embodiment of the invention, the stream comprising two or more alcohols is an aqueous stream comprising the reaction product stream from a process for the hydrogenolysis of a saccharide-containing feedstock.

The two or more alcohols are glycols. In the embodiment wherein the stream comprising two or more alcohols is an aqueous stream comprising the reaction product stream from a process for the hydrogenolysis of a saccharide-containing feedstock, the alcohols are selected from the group consisting of MEG, MPG and 1,2-BDO. These alcohols are typically present at a concentration in the range of from 0.1 to 30 wt%.

As well as the two alcohols, the reaction product streams from hydrogenolysis reactions of saccharides may comprise water, oxygenates, hydrocarbons, catalyst, degradation products, and gases in any composition. The variety of compounds and their concentration depend on the saccharide-containing feedstock and the various hydrogenation and hydrogenolysis conversion conditions, including catalysts, reaction conditions such as temperature, pressure and saccharide concentration.

Water must be present in the process of the present invention. This water may be present as part of the stream comprising two or more alcohols. Optionally, a further aqueous stream is added to the process. Said further aqueous stream preferably consists essentially of water. Alternatively, said further aqueous stream may comprise at least in part of a recycle stream from the process of the present invention, said recycle stream comprising water and, optionally, one or more alcohols.

The solvent comprises an alkyl amine. More preferably, the solvent comprises a primary, a secondary, a tertiary alkyl amine, or a combination thereof. Preferably the amine is a tertiary alkyl amine. Examples of suitable alkyl amines include paraffinic amines, naphthenic amines, aromatic amines, and mixtures thereof. Suitable alkyl amines include any amines that show a liquid-liquid phase split when mixed with water or saline water at appropriate process temperatures, preferably in the range of from 0 to 250°C.

Preferably, the amine contains carbon and nitrogen atoms in a ratio of at most 8:1 (carbon:nitrogen atoms).

Preferably, the amine contains an aliphatic cyclic group either containing the amine nitrogen or attached to the amine nitrogen.
More preferably, the solvent is selected from the group consisting of N,N-dimethylcyclohexylamine (DMCA), methyl cyclohexyl amine, N-methyl piperidine, triethylamine, tripropylamine, or a combination thereof.

The solvent may be added to or combined with the stream comprising two or more alcohols in any amount sufficient to allow a portion of the first alcohol to dissolve in the solvent. Water may also dissolve in the solvent to the extent that the alcohol to water ratio in the extract stream is larger than in the stream comprising two or more alcohols. In certain embodiments, the amount of solvent added to or combined with the stream comprising two or more alcohols may be from 10 to 500wt% of the total content of that stream.

Preferably, the ratio of solvent to alcohol may be the minimum amount for exceeding the solubility limit of the solvent in the product stream to less than the amount needed to dissolve the entire feed stream. The amount of solvent added to or combined with the stream comprising two or more alcohols may suitably be at least 10wt%, preferably at least 20wt%, more preferably at least 40wt% of the amount of alcohol in the stream comprising two or more alcohols. The amount of solvent added to or combined with the stream comprising two or more alcohols may suitably be at most 2000wt%, preferably at most 500wt%, more preferably at most 100wt% of the amount of alcohol in the stream comprising two or more alcohols.

The stream comprising two or more alcohols is combined with the solvent stream by any method suitable for the combination of two liquid streams, including but not limited to using a stirred mixer, passing the streams through a static mixer or by agitation. State of the art liquid-liquid contactors (extraction units) are, for example, a series of mixers and settlers, agitated extraction columns, packed extraction columns, SCHEIBEL® Columns, KARR® Columns, rotating disc contactor (RDC) columns, pulsed, packed (SMVP) and sieve tray columns. In a preferred embodiment of the invention, the two streams are combined in a counter-current extraction unit. In such a unit, the two streams are fed to the unit at points separated by at least 50% of the length, preferably substantially the entire length, of the unit and are brought into contact with each other while passing through the unit in a counter-current fashion.

The first alcohol is recovered from the stream comprising two or more alcohols by liquid-liquid extraction after the solvent has been added to or combined with the stream comprising two or more alcohols. For example, after the solvent has been added to or combined with the stream comprising two or more alcohols, a portion of the alcohol may be extracted into the solvent. The solvent, along with the alcohol, may then be separated from the rest of the stream comprising two or more alcohols forming a first alcohol and solvent rich stream and a first residual stream.

Preferably, any salt remains dissolved in the stream comprising two or more alcohols so that the separation process happens without precipitation of salts.

In certain embodiments, the liquid-liquid extraction may be enhanced by the inclusion of a synergist. Examples of suitable synergist include demulsifiers. Typical demulsifiers can be phenol-formaldehyde resins, epoxy resins, polyamines, di-epoxides or polyols.

Preferably, the method further comprises recovering the first alcohol and/or solvent from the first alcohol and solvent rich stream. The first alcohol and/or solvent may be recovered from the first alcohol and solvent rich stream through a distillation process. In certain embodiments, the solvent or the first alcohol may be recovered as the distillate or bottom product. In certain embodiments, the first alcohol and solvent rich stream may be distilled to form a first alcohol rich stream and a solvent rich stream. Optionally, the solvent may be recycled.

In a preferred embodiment of the invention, after recovering a first alcohol according to the process of the invention, a second alcohol is subsequently recovered from the first residual stream by a process comprising the steps of providing the first residual stream, providing a solvent stream, combining said first residual stream with said solvent stream and recovering at least a portion of the second alcohol by liquid-liquid extraction.

The solvent used in the recovery of the second alcohol may be the same or different to the solvent used in the recovery of the first alcohol. As with the solvent used in the recovery of the first alcohol, the solvent comprises a solvent that has a higher affinity for alcohol than water. Preferably, the solvent comprises an alkyl amine. More preferably, the solvent comprises a primary, a secondary, a tertiary alkyl amine, or a combination thereof. Preferably the amine is a tertiary alkyl amine. Examples of suitable alkyl amines include paraffinic amines, naphthenic amines, aromatic amines, and mixtures thereof. Suitable alkyl amines include any amines that show a liquid-liquid phase split when mixed with water or saline water at appropriate process temperatures, preferably in the range of from 0 to 250°C.

Preferably, the amine contains carbon and nitrogen atoms in a ratio of at most 8:1 (carbon:nitrogen atoms).

Preferably, the amine contains an aliphatic cyclic group either containing the amine nitrogen or attached to the amine nitrogen.

More preferably, the solvent is selected from the group consisting of N,N-dimethylcyclohexylamine (DMCA), methyl cyclohexyl amine, N-methyl piperidine, triethylamine, tripropylamine, or a combination thereof.

The solvent may be added to or combined with the stream comprising two or more alcohols in any amount sufficient to allow a portion of the second alcohol to dissolve in the solvent. Water may also dissolve in the solvent to the extent that the alcohol to water ratio in the extract stream is larger than in the first residual stream. In certain embodiments, the amount of solvent added to or combined with the first residual stream may be from 10 to 500wt% of the total content of that stream.

Preferably, the ratio of solvent to alcohol may be the minimum amount for exceeding the solubility limit of the solvent in the product stream to the amount needed to dissolve the entire feed stream. The amount of solvent added to or combined with the stream comprising two or more alcohols may suitably be at least 10wt%, preferably at least 20wt%, more preferably at least 40wt% of the amount of alcohol in the stream comprising two or more alcohols. The amount of solvent added to or combined with the stream comprising two or more alcohols may suitably be at most 2000wt%, preferably at most 100wt%, more preferably at most 75wt% of the amount of alcohol in the stream comprising two or more alcohols.

The first residual stream is combined with the solvent stream by any method suitable for the combination of two liquid streams, including but not limited to using a stirred mixer, passing the streams through a static mixer or by agitation. State of the art liquid-liquid contactors (extraction units) are, for example, a series of mixers and settlers, agitated extraction columns, packed extraction columns, SCHEIBEL® Columns, KARR® Columns, rotating disc contactor (RDC) columns, pulsed, packed (SMVP) and sieve tray columns. In a preferred embodiment of the invention, the two streams are combined in a counter-current extraction unit. In such a unit, the two streams are fed to the unit at points separated by at least 50% of the length, preferably substantially the entire length, of the unit and are brought into contact with each other while passing through the unit in a counter-current fashion.

The second alcohol is recovered from the first residual stream by liquid-liquid extraction after the solvent has been added to or combined with the first residual stream. For example, after the solvent has been added to or combined with the first residual stream, a portion of the second alcohol may be extracted into the solvent. The solvent, along with the second alcohol, may then be separated from the rest of the first residual stream forming a second alcohol and solvent rich stream and a second residual stream.

Preferably, any salt remains dissolved in the first residual stream so that the separation process happens without precipitation of salts.

In certain embodiments, the liquid-liquid extraction may be enhanced by the inclusion of a synergist. Examples of suitable synergist include demulsifiers. Typical demulsifiers can be phenol-formaldehyde resins, epoxy resins, polyamines, di-epoxides or polyols.

Preferably, the method further comprises recovering the second alcohol and/or solvent from the second alcohol and solvent rich stream. The second alcohol and/or solvent may be recovered from the second alcohol and solvent rich stream through a distillation process. In certain embodiments, the solvent or the second alcohol may be recovered as the distillate or bottom product. In certain embodiments, the second alcohol and solvent rich stream may be distilled to form a second alcohol rich stream and a solvent rich stream. Optionally, the solvent may be recycled.

In a preferred embodiment of the invention, the solvent used in the recovery of the second alcohol is the same as the solvent used in the recovery of the first alcohol. In this embodiment it is particularly advantageous to use a solvent which is more selective to the first alcohol than the second.

In a further preferred embodiment of the invention, after recovering a second alcohol according to the process of the invention, a third alcohol is subsequently recovered from the second residual stream by a process comprising the steps of providing the second residual stream, providing a solvent stream, combining said second residual stream with said solvent stream and recovering at least a portion of the third alcohol by liquid-liquid extraction.

The solvent used in the recovery of the third alcohol may be the same or different to the solvent used in the recovery of the first alcohol and may also be the same or different from the solvent used in the recovery of the second alcohol. The solvent used in the recovery of the third alcohol suitably comprises a solvent that has a higher affinity for alcohol than water. Preferably, the solvent comprises an alkyl amine. More preferably, the solvent comprises a primary, a secondary, a tertiary alkyl amine, or a combination thereof. Preferably the amine is a tertiary alkyl amine. Examples of suitable alkyl amines include paraffinic amines, naphthenic amines, aromatic amines, and mixtures thereof. Suitable alkyl amines include any amines that show a liquid-liquid phase split when mixed with water or saline water at appropriate process temperatures, preferably in the range of from 0 to 250°C.

Preferably, the amine contains carbon and nitrogen atoms in a ratio of at most 8:1 (carbon:nitrogen atoms).

Preferably, the amine contains an aliphatic cyclic group either containing the amine nitrogen or attached to the amine nitrogen.

More preferably, the solvent is selected from the group consisting of N,N-dimethylcyclohexylamine (DMCA), methyl cyclohexyl amine, N-methyl piperidine, triethylamine, tripropylamine, or a combination thereof.

The solvent may be added to or combined with the second residual stream in any amount sufficient to allow a portion of the third alcohol to dissolve in the solvent. Water may also dissolve in the solvent to the extent that the third alcohol to water ratio in the extract stream is larger than in the second residual stream. In certain embodiments, the amount of solvent added to or combined with the second residual stream may be from 10 to 500wt% of the total content of that stream.

Preferably, the ratio of solvent to alcohol may be the minimum amount for exceeding the solubility limit of the solvent in the product stream to the amount needed to dissolve the entire feed stream. The amount of solvent added to or combined with the stream comprising two or more alcohols may suitably be at least 10wt%, preferably at least 20wt%, more preferably at least 40wt% of the amount of alcohol in the stream comprising two or more alcohols. The amount of solvent added to or combined with the stream comprising two or more alcohols may suitably be at most 2000wt%, preferably at most 100wt%, more preferably at most 75wt% of the amount of alcohol in the stream comprising two or more alcohols.

The second residual stream is combined with the solvent stream by any method suitable for the combination of two liquid streams, including but not limited to using a stirred mixer, passing the streams through a static mixer or by agitation. State of the art liquid-liquid contactors (extraction units) are, for example, a series of mixers and settlers, agitated extraction columns, packed extraction columns, SCHEIBEL® Columns, KARR® Columns, rotating disc contactor (RDC) columns, pulsed, packed (SMVP) and sieve tray columns. In a preferred embodiment of the invention, the two streams are combined in a counter-current extraction unit. In such a unit, the two streams are fed to the unit at points separated by at least 50% of the length, preferably substantially the entire length, of the unit and are brought into contact with each other while passing through the unit in a counter-current fashion.

The third alcohol is recovered from the second residual stream by liquid-liquid extraction after the solvent has been added to or combined with the second residual stream. For example, after the solvent has been added to or combined with the second residual stream, a portion of the third alcohol may be extracted into the solvent. The solvent, along with the third alcohol, may then be separated from the rest of the second residual stream forming a third alcohol and solvent rich stream and a third residual stream.

Preferably, any salt remains dissolved in the second residual stream so that the separation process happens without precipitation of salts.

In certain embodiments, the liquid-liquid extraction may be enhanced by the inclusion of a synergist. Examples of suitable synergist include demulsifiers. Typical demulsifiers can be phenol-formaldehyde resins, epoxy resins, polyamines, di-epoxides or polyols.

Preferably, the method further comprises recovering the third alcohol and/or solvent from the third alcohol and solvent rich stream. The third alcohol and/or solvent may be recovered from the third alcohol and solvent rich stream through a distillation process. In certain embodiments, the solvent or the third alcohol may be recovered as the distillate or bottom product. In certain embodiments, the third alcohol and solvent rich stream may be distilled to form a third alcohol rich stream and a solvent rich stream. Optionally, the solvent may be recycled.

In a preferred embodiment of the invention, the solvent used in the recovery of the third alcohol is the same as the solvent used in the recovery of the first alcohol and the solvent used in the recovery of the second alcohol. In this embodiment it is particularly advantageous to use a solvent which is more selective to the first alcohol than the second or third alcohols and more selective to the second alcohol than the third alcohol. An example of a solvent that is more selective to one type of alcohol than to another is dimethylcyclohexylamine, which has been shown to be more than five times more selective to 1,2-butanediol than to monoethylene glycol.

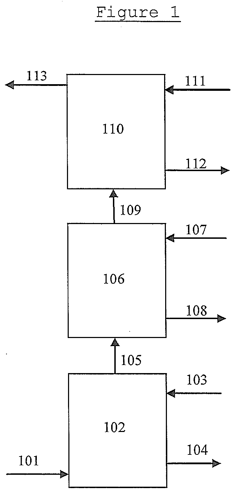

In a particularly preferred, but non-limiting, embodiment of the invention illustrated in Figure 1, the stream comprising two or more alcohols comprises the reaction product stream from a process for the hydrogenolysis of a saccharide-containing feedstock. The stream contains at least MEG, MPG and 1,2-BDO as alcohols. This stream 101 is fed into a counter-current extraction unit comprising three extraction stages. The stream 101 is introduced to a first extraction stage 102, where it is contacted in a counter-current manner with a first solvent stream 103. A first alcohol and solvent rich stream 104 is removed and a first residual stream 105 is passed to a second extraction stage 106. In the second extraction stage 106, the first residual stream is contacted in a counter-current manner with a second solvent stream 107. A second alcohol and solvent rich stream 108 is removed and a second residual stream 109 is passed to a third extraction stage 110. In the third extraction stage 110, the second residual stream is contacted in a counter-current manner with a third solvent stream 111. A third alcohol and solvent rich stream 112 is removed and a third residual stream 113 is also removed for further purification, recycle or disposal. In this embodiment, the first alcohol comprises 1,2-BDO, the second alcohol comprises MPG and the third alcohol comprises MEG.

Each alcohol and solvent rich stream can be separately treated to provide the alcohol and the solvent, suitably by distillation. It will be readily understood by the skilled person that each extraction stage may incorporate multiple steps of adding a solvent stream, contacting it with the stream comprising two or more alcohols or the residual stream and separating an alcohol and solvent rich stream. Further, the individual extraction stages may be in separate vessels or may be contained within a single vessel.

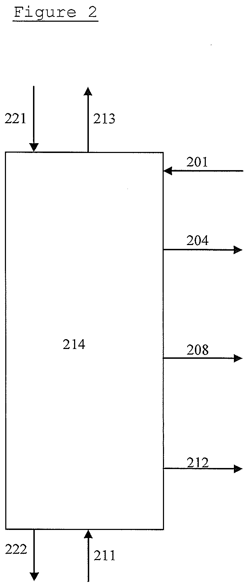

In a further preferred, but non-limiting, embodiment illustrated in Figure 2, the stream comprising two or more alcohols comprises the reaction product stream from a process for the hydrogenolysis of a saccharide-containing feedstock. The stream contains at least MEG, MPG and 1,2-BDO as alcohols. This stream 201 is fed into a counter-current extraction unit 214. Said extraction unit is also fed with solvent stream 211, which is contacted with stream 201 in a counter-current manner. A first alcohol and solvent rich stream 204 is removed. Subsequently, a second alcohol and solvent rich stream 208 and a third alcohol and solvent rich stream 212 are removed. A residual stream 222 is also removed for further purification, recycle or disposal.

Optionally, an aqueous stream 221 may be added to the extraction unit 214. The residual stream 222 may then be recycled and added to aqueous stream 221 before it is fed into extraction unit 214. In this case, the feed stream 201 may be located at any height (tray) in extraction unit 214, even below the first 204 or second 208 alcohol rich streams.

Typically the process of the invention is carried out at temperatures in the range of from 0 to 200°C. The temperature may be altered in order to tailor the process to be specific to specific alcohols. Further, in the embodiment of the invention in which a second and, optionally, a third alcohol are subsequently recovered it is preferred that the temperature is altered such that the recovery of each alcohol is carried out at a different temperature. In a particularly preferred embodiment of the invention, wherein the first alcohol is 1,2-BDO and the second or third alcohol is MEG, a lower temperature is used during the recovery of 1,2-BDO than is used during the recovery of MEG. In a preferred embodiment of the invention illustrated in Figure 2, the temperature is reduced along the extraction unit 214, such that the temperature is highest at the point of addition of the aqueous stream 221 and lowest at the point of addition of the stream 211.

The temperature profile, if present, and the ratio of feed stream 201 flow to solvent stream 211 flow to aqueous stream 221 flow (if present) will provide a composition profile of alcohols that is unique for each different alcohol and can be used to define the optimum location of the feed stream 201 tray and alcohol rich streams (204, 208 and 212) trays.

The present invention is further illustrated in the following Examples.

### Examples

In each of the following examples, where relevant, to calculate the selectivity, the ratio of the concentration in the upper phase over the concentration in the lower phase was calculated (K-values). The solvent DMCA is the key component of the upper phase. The selectivity of the solvent for one component compared to a second component is the ratio of the K-value of the first component over the K-value of the second component.

### Example 1

A solution of 10 wt% MEG in water was mixed with DMCA in a weight ratio of 1:1. This mixture was stirred at 50°C for 30 minutes and after stopping the stirring two liquid phases quickly separated. Samples were taken from both liquid phases and analyzed by gas chromatography.

The selectivity of DMCA for MEG over water was 1.53 at 50°C.

### Example 2

A solution of 10 wt% 1,2-BDO in water was mixed with DMCA in a weight ratio of 1:1. This mixture was stirred at 50°C for 30 minutes and after stopping the stirring two liquid phases quickly separated. Samples were taken from both liquid phases and analyzed by gas chromatography.

The selectivity of DMCA for 1,2-BDO over water was 8.20 at 50°C.

### Example 3

The results of Examples 1 and 2 were combined to provide a selectivity of 1,2-BDO over MEG was 5.36 at 50°C.

### Example 4

A solution of 10 wt% MEG in water was mixed with DMCA in a weight ratio of 1:1. This mixture was stirred at 20°C for 30 minutes and after stopping the stirring two liquid phases quickly separated. Samples were taken from both liquid phases and analyzed by gas chromatography.

The selectivity of DMCA for MEG over water was 1.23 at 20°C.

### Example 5

A solution of 10 wt% 1,2-BDO in water was mixed with DMCA in a weight ratio of 1:1. This mixture was stirred at 20°C for 30 minutes and after stopping the stirring two liquid phases quickly separated. Samples were taken from both liquid phases and analyzed by gas chromatography.

The selectivity of DMCA for 1,2-BDO over water was 3.93 at 20°C.

### Example 6

The results of Examples 4 and 5 were combined to provide a selectivity of 1,2-BDO over MEG was 3.20 at 20°C.

### Example 7

A solution of 10 wt% MEG in water was mixed with DMCA in a weight ratio of 1:1. This mixture was stirred at 90°C for 30 minutes and after stopping the stirring two liquid phases quickly separated. Samples were taken from both liquid phases and analyzed by gas chromatography.

The selectivity of DMCA for MEG over water was 2.10 at 90°C.

### Example 8

A solution of 10 wt% 1,2-BDO in water was mixed with DMCA in a weight ratio of 1:1. This mixture was stirred at 90°C for 30 minutes and after stopping the stirring two liquid phases quickly separated. Samples were taken from both liquid phases and analyzed by gas chromatography.

The selectivity of DMCA for 1,2-BDO over water was 13.22 at 90°C.

### Example 9

The results of Examples 7 and 8 were combined to provide a selectivity of 1,2-BDO over MEG was 6.30 at 90°C.

### Example 10

A solution of 10 wt% MPG in water was mixed with DMCA in a weight ratio of 1:1. This mixture was stirred at 50°C for 30 minutes and after stopping the stirring two liquid phases quickly separated. Samples were taken from both liquid phases and analyzed by gas chromatography.

The selectivity of DMCA for MPG over water was 3.10 at 50°C.

### Example 11

The results of Examples 1 and 10 were combined to provide a selectivity of MPG over MEG was 2.03 at 50°C.

### Example 12

The results of Examples 2 and 12 were combined to provide a selectivity of 1,2-BDO over MPG was 2.65 at 50°C.

### Example 13

A solution of 10wt% MPG in water was mixed with DMCA in a weight ratio of 1:1. This mixture was stirred at 20°C for 30 minutes and after stopping the stirring two liquid phases quickly separated. Samples were taken from both liquid phases and analyzed by gas chromatography.

The selectivity of DMCA for MPG over water was 1.84 at 20°C.

### Example 14

The results of Examples 4 and 13 were combined to provide a selectivity of MPG over MEG was 1.50 at 20°C.

### Example 15

The results of Examples 5 and 13 were combined to provide a selectivity of 1,2-BDO over MPG was 2.14 at 20°C.

### Example 16

A solution of 10 wt% MPG in water was mixed with DMCA in a weight ratio of 1:1. This mixture was stirred at 90°C for 30 minutes and after stopping the stirring two liquid phases quickly separated. Samples were taken from both liquid phases and analyzed by gas chromatography.

The selectivity of DMCA for MPG over water was 5.26 at 90 °C.

### Example 17

The results of Examples 7 and 16 were combined to provide a selectivity of MPG over MEG was 2.50 at 90 °C.

### Example 18

The results of Examples 8 and 16 were combined to provide a selectivity of 1,2-BDO over MPG was 2.51 at 90 °C.

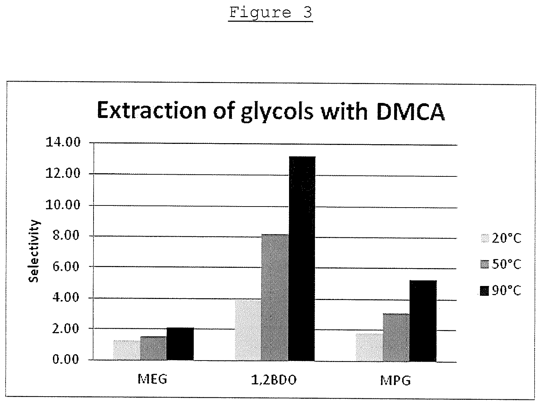

Figure 3 illustrates the selectivities of glycols over water at three temperatures, 20, 50 and 90°C. Both the selectivities of glycols over water and the selectivities of glycols with a higher molecular weight over glycols with a lower molecular weight are higher at 90°C than at 50°C than at 20°C.

### Example 19

A solution of 10 wt% MEG in water was mixed with DMCA in a weight ratio of 2:1 (aqueous solution:DMCA). This mixture was stirred at 50°C for 30 minutes and after stopping the stirring two liquid phases quickly separated. Samples were taken from both liquid phases and analyzed by gas chromatography.

The selectivity of DMCA for MEG over water was 1.03 at 50°C.

### Example 20

A solution of 10 wt% 1,2-BDO in water was mixed with DMCA in a weight ratio of 2:1 (aqueous solution:DMCA). This mixture was stirred at 50°C for 30 minutes and after stopping the stirring two liquid phases quickly separated. Samples were taken from both liquid phases and analyzed by gas chromatography.

The selectivity of DMCA for 1,2-BDO over water was 7.02 at 50°C.

### Example 21

A solution of 10 wt% 1,2-BDO in water was mixed with DMCA in a weight ratio of 10:1 (aqueous solution:DMCA). This mixture was stirred at 50°C for 30 minutes and after stopping the stirring two liquid phases quickly separated. Samples were taken from both liquid phases and analyzed by gas chromatography.

The selectivity of DMCA for 1,2-BDO over water was 6.65 at 50°C.

### Example 22

The results of Examples 2, 20 and 21 were combined to show that the selectivity of 1,2-BDO over water is maintained at a high level above 6.5 even when the weight amount of solvent is only one tenth of the aqueous glycol mixture.

### Example 23

A solution of 9 wt% MEG and 1 wt% 1,2-BDO in water was mixed with DMCA in a weight ratio of 10:1 (aqueous solution:DMCA). This mixture was stirred at 50°C for 30 minutes and after stopping the stirring two liquid phases quickly separated. Samples were taken from both liquid phases and analyzed by gas chromatography.

The selectivity of DMCA for MEG over water was 1.67 at 50°C, in line with 4).

The selectivity of DMCA for 1,2-BDO over water was 10.595 at 50 °C.

The selectivity of DMCA for 1,2-BDO over MEG was 6.36 at 50°C.

## Claims

1. A process for the recovery of a first glycol from a stream comprising two or more glycols, said process comprising the steps of providing a stream comprising two or more glycols, providing a solvent stream, combining said stream comprising two or more glycols with said solvent stream in the presence of water and recovering at least a portion of the first glycol by liquid-liquid extraction, wherein the first glycol is recovered from the solvent of the solvent stream and the solvent comprises an alkylamine.

2. A process according to claim 1, wherein the stream comprising two or more glycols is an aqueous stream.

3. A process according to claim 1 or claim 2, wherein water is added to the process as a further aqueous stream.

4. A process according to any one of claims 1 to 3, wherein at least a portion of the first glycol is recovered by liquid-liquid extraction by a process comprising the steps of extracting a portion of the first glycol into the solvent and separating a first glycol and solvent rich stream, leaving a first residual stream.

5. A process according to claim 4, wherein a second glycol is subsequently recovered from the first residual stream by a process comprising the steps of providing the first residual stream, providing a solvent stream, combining said first residual stream with said solvent stream and recovering at least a portion of the second glycol from the solvent of the solvent stream by liquid-liquid extraction.

6. A process according to claim 5, wherein at least a portion of the second glycol is recovered by liquid-liquid extraction by a process comprising the steps of extracting a portion of the second glycol into the solvent and separating a second glycol and solvent rich stream, leaving a second residual stream.

7. A process according to claim 6, wherein a third glycol is subsequently recovered from the second residual stream by a process comprising the steps of providing the second residual stream, providing a solvent stream, combining said second residual stream with said solvent stream and recovering at least a portion of the third glycol from the solvent of the solvent stream by liquid-liquid extraction.

8. A process according to claim 7, wherein at least a portion of the third glycol is recovered by liquid-liquid extraction by a process comprising the steps of extracting a portion of the third glycol into the solvent and separating a third glycol and solvent rich stream, leaving a third residual stream.

9. A process according to any one of claims 4, 6 or 8, wherein the glycol and solvent rich stream is then separated into a glycol rich stream and a solvent rich stream by distillation.

10. A process according to any one of claims 1 to 9, wherein the first glycol is 1,2-butanediol.

11. A process according to any one of claims 5 to 10, wherein the second glycol is selected from monopropylene glycol and monoethylene glycol.

12. A process according to any one of claims 7 to 11, wherein the second glycol is monopropylene glycol and the third glycol is monoethylene glycol.

13. A process according to any one of claims 1 to 12, wherein the solvent is selected from the group consisting of dimethyl-cyclohexyl amine, methyl cyclohexyl amine, 1-methyl piperidine, triethylamine, tripropylamine, or a combination thereof.

14. A process according to any one of claims 5 to 13, wherein the second glycol is recovered at a lower temperature than the first glycol.

15. A process according to any one of claims 7 to 14, wherein the third glycol is recovered at a lower temperature than the second glycol.

## Patentansprüche

1. Verfahren zur Rückgewinnung eines ersten Glycols aus einem Strom, der zwei oder mehr Glycole umfasst, wobei das Verfahren die Schritte des Bereitstellens eines Stroms umfassend zwei oder mehr Glycole, des Bereitstellens eines Lösungsmittelstroms, des Vereinigens des Stroms umfassend zwei oder mehr Glycole mit dem Lösungsmittelstrom in Gegenwart von Wasser und des Rückgewinnens mindestens eines Teils des ersten Glycols durch Flüssig-Flüssig-Extraktion umfasst, wobei das erste Glycol aus dem Lösungsmittel des Lösungsmittelstroms rückgewonnen wird und das Lösungsmittel ein Alkylamin umfasst.

2. Verfahren nach Anspruch 1, wobei der Strom umfassend zwei oder mehr Glycole ein wässriger Strom ist.

3. Verfahren nach Anspruch 1 oder Anspruch 2, wobei dem Verfahren als weiterer wässriger Strom Wasser zugegeben wird.

4. Verfahren nach einem der Ansprüche 1 bis 3, wobei mindestens ein Teil des ersten Glycols durch Flüssig-Flüssig-Extraktion durch ein Verfahren rückgewonnen wird, welches die Schritte des Extrahierens eines Teils des ersten Glycols in das Lösungsmittel und des Abscheidens eines ersten Glycols und eines lösungsmittelreichen Stroms umfasst, wobei einer erster Reststrom zurückbleibt.

5. Verfahren nach Anspruch 4, wobei ein zweites Glycol anschließend aus dem ersten Reststrom durch ein Verfahren rückgewonnen wird, das die Schritte des Bereitstellens des ersten Reststroms, des Bereitstellens eines Lösungsmittelstroms, des Vereinigens des ersten Reststroms mit dem Lösungsmittelstrom und des Rückgewinnens von mindestens einem Teil des zweiten Glycols aus dem Lösungsmittel des Lösungsmittelstroms durch Flüssig-Flüssig-Extraktion umfasst.

6. Verfahren nach Anspruch 5, wobei mindestens ein Teil des zweiten Glycols durch Flüssig-Flüssig-Extraktion durch ein Verfahren rückgewonnen wird, welches die Schritte des Extrahierens eines Teils des zweiten Glycols in das Lösungsmittel und des Abscheidens eines zweiten Glycols und eines lösungsmittelreichen Stroms umfasst, wobei einer zweiter Reststrom zurückbleibt.

7. Verfahren nach Anspruch 6, wobei ein drittes Glycol anschließend aus dem zweiten Reststrom durch ein Verfahren rückgewonnen wird, das die Schritte des Bereitstellens des zweiten Reststroms, des Bereitstellens eines Lösungsmittelstroms, des Vereinigens des zweiten Reststroms mit dem Lösungsmittelstrom und des Rückgewinnens von mindestens einem Teil des dritten Glycols aus dem Lösungsmittel des Lösungsmittelstroms durch Flüssig-Flüssig-Extraktion umfasst.

8. Verfahren nach Anspruch 7, wobei mindestens ein Teil des dritten Glycols durch Flüssig-Flüssig-Extraktion durch ein Verfahren rückgewonnen wird, welches die Schritte des Extrahierens eines Teils des dritten Glycols in das Lösungsmittel und des Abscheidens eines dritten Glycols und eines lösungsmittelreichen Stroms umfasst, wobei einer dritter Reststrom zurückbleibt.

9. Verfahren nach einem der Ansprüche 4, 6 oder 8, wobei der glycol- und lösungsmittelreiche Strom dann durch Destillation in einen glycolreichen Strom und einen lösungsmittelreichen Strom abgeschieden wird.

10. Verfahren nach einem der Ansprüche 1 bis 9, wobei das erste Glycol 1,2-Butandiol ist.

11. Verfahren nach einem der Ansprüche 5 bis 10, wobei das zweite Glycol aus Monopropylenglycol und Monoethylenglycol ausgewählt wird.

12. Verfahren nach einem der Ansprüche 7 bis 11, wobei es sich bei dem zweiten Glycol um Monopropylenglycol und bei dem dritten Glycol um Monoethylenglycol handelt.

13. Verfahren nach einem der Ansprüche 1 bis 12, wobei das Lösungsmittel aus der Gruppe bestehend aus Dimethylcyclohexylamin, Methylcyclohexylamin, 1-Methylpiperidin, Triethylamin, Tripropylamin oder einer Kombination davon ausgewählt wird.

14. Verfahren nach einem der Ansprüche 5 bis 13, wobei das zweite Glycol bei einer niedrigeren Temperatur rückgewonnen wird als das erste Glycol.

15. Verfahren nach einem der Ansprüche 7 bis 14, wobei das dritte Glycol bei einer niedrigeren Temperatur rückgewonnen wird als das zweite Glycol.

## Revendications

1. Procédé de récupération d'un premier glycol à partir d'un courant comprenant deux glycols ou plus, ledit procédé comprenant les étapes de fourniture d'un courant comprenant deux glycols ou plus, de fourniture d'un courant de solvant, de combinaison dudit courant comprenant deux glycols ou plus avec ledit courant de solvant en présence d'eau et de récupération d'au moins une partie du premier glycol par extraction liquide-liquide, le premier glycol étant récupéré à partir du solvant du courant de solvant et le solvant comprenant une alkylamine.

2. Procédé selon la revendication 1, dans lequel le courant comprenant deux glycols ou plus est un courant aqueux.

3. Procédé selon la revendication 1 ou la revendication 2, dans lequel de l'eau est ajoutée au procédé comme courant aqueux supplémentaire.

4. Procédé selon l'une quelconque des revendications 1 à 3, dans lequel au moins une partie du premier glycol est récupérée par extraction liquide-liquide par un procédé comprenant les étapes d'extraction d'une partie du premier glycol dans le solvant et de séparation d'un premier courant riche en glycol et en solvant, laissant un premier courant résiduel.

5. Procédé selon la revendication 4, dans lequel un deuxième glycol est ensuite récupéré à partir du premier courant résiduel par un procédé comprenant les étapes de fourniture du premier courant résiduel, de fourniture d'un courant de solvant, de combinaison dudit premier courant résiduel avec ledit courant de solvant et de récupération d'au moins une partie du deuxième glycol à partir du solvant du courant de solvant par extraction liquide-liquide.

6. Procédé selon la revendication 5, dans lequel au moins une partie du deuxième glycol est récupérée par extraction liquide-liquide par un procédé comprenant les étapes d'extraction d'une partie du deuxième glycol dans le solvant et de séparation d'un deuxième courant riche en glycol et en solvant, laissant un deuxième courant résiduel.

7. Procédé selon la revendication 6, dans lequel un troisième glycol est ensuite récupéré à partir du deuxième courant résiduel par un procédé comprenant les étapes de fourniture du deuxième courant résiduel, de fourniture d'un courant de solvant, de combinaison dudit deuxième courant résiduel avec ledit courant de solvant et de récupération d'au moins une partie du troisième glycol à partir du solvant du courant de solvant par extraction liquide-liquide.

8. Procédé selon la revendication 7, dans lequel au moins une partie du troisième glycol est récupérée par extraction liquide-liquide par un procédé comprenant les étapes d'extraction d'une partie du troisième glycol dans le solvant et de séparation d'un troisième courant riche en glycol et en solvant, laissant un troisième courant résiduel.

9. Procédé selon l'une quelconque des revendications 4, 6 ou 8, dans lequel le courant riche en glycol et en solvant est ensuite séparé en un courant riche en glycol et en un courant riche en solvant par distillation.

10. Procédé selon l'une quelconque des revendications 1 à 9, dans lequel le premier glycol est le 1,2-butanediol.

11. Procédé selon l'une quelconque des revendications 5 à 10, dans lequel le deuxième glycol est choisi parmi le monopropylène glycol et le monoéthylène glycol.

12. Procédé selon l'une quelconque des revendications 7 à 11, dans lequel le deuxième glycol est le monopropylène glycol et le troisième glycol est le monoéthylène glycol.

13. Procédé selon l'une quelconque des revendications 1 à 12, dans lequel le solvant est choisi dans le groupe comprenant la diméthylcyclohexylamine, la méthylcyclohexylamine, la 1-méthylpipéridine, la triéthylamine, la tripropylamine ou une combinaison de ces dernières.

14. Procédé selon l'une quelconque des revendications 5 à 13, dans lequel le deuxième glycol est récupéré à une température inférieure à celle du premier glycol.

15. Procédé selon l'une quelconque des revendications 7 à 14, dans lequel le troisième glycol est récupéré à une température inférieure à celle du deuxième glycol.
